# EUROPEAN PATENT SPECIFICATION

(11) **EP 3 169 371 B1**
(45) Date of publication and mention of the grant of the patent: **12.05.2021**
(21) Application number: 15804023.8
(22) Date of filing: 02.03.2015
(51) Int. Cl.: A61L 15/28, C08L 3/04, A61L 24/00, A61L 24/04, C08J 3/00, C08J 5/18, C08L 3/02

(54) **SURGICAL HEMOSTATIC BASED RICE STARCH**
AUF REISSTÄRKE BASIERENDE CHIRURGISCHES HÄMOSTATIKUM
MATÉRIAU HÉMOSTATIQUE CHIRURGICAL À BASE D'AMIDON DE RIZ

(30) Priority: 21.05.2014 TH 1401002984
(43) Date of publication of application: 24.05.2017
(73) Proprietor: Punyanitya, Sittiporn, Chiang Mai 50000 (TH); Ruksanti, Anucha, Chiang Mai 50130 (TH)
(72) Inventor: Punyanitya, Sittiporn, Chiang Mai 50000 (TH); Ruksanti, Anucha, Chiang Mai 50130 (TH)
(74) Representative: Jeck, Anton
(86) International application number: PCT/TH2015/000014
(87) International publication number: WO 2015/187103

(56) References cited:
- EP-A1- 2 233 157
- WO-A1-2009/076873
- CN-A- 1 554 448
- CN-A- 102 286 184
- US-A1- 2007 276 345
- US-A1- 2009 062 233
- US-B2- 7 604 819

## Description

### TECHNICAL FIELD

Polymer chemistry

### BACKGROUND OF THE INVENTION

Hemostatic while surgery operations such as a brain surgery can notstopped bleeding by a vascular restraining. Surgeon will use heat or chemical materials to stop bleeding. The principle for stop bleeding by chemical materials, for example, is blood adsorption into the material until the compressive force is high enough to block the bleeding area. After that, the bleeding site will naturally clotted and finally stopped, respectively.

In 1945, Correll and Wise had prepared a gelatin sponge from collagen for using in surgery and it is wide used until present. The gelatin sponge is enormously adsorbed fluid and then it has compressive pressure to press at bleeding area, and it is biocompatible biomaterials within living tissue, thus it does not required removal *in vivo* EP 2 233 157 A1 discloses a composition for preparing a surgical hemostatic starch-based film. The composition comprises starch which can be processed by freeze drying and other components, such as carboxymethyl cellulose and glycerol can be added.

A natural polymer is an inexpensive polymer, biocompatible and also biodegradable. It can be prepared for cell culture in tissue engineering and also applied for drug delivery system.

All natural polymers, especially rice starch powder is interested due to the biological useful properties. Starch is a glucan, a polysaccharide consisting of glucose unit, which comprised two types that is amylose and amylopectin. It is synthesized by plant as a source of food reserves and accumulated in cell cytoplasm in granules. However, starch has some disadvantages such as high hydrophilic property that can be immediately degradable and poor mechanical property, especially in a wet condition. Thus starch modification must be improved before use as raw material.

Rice starch is major food for people of East Asia, Southeast Asia, and also South Asia, and about 90% of rice is produced and consumed in these areas. The main component of milled rice is composed of 79.2% carbohydrate, 7.0% protein, 0.4% fat, and 0.5% ash.

The rice starch application for using as a hemostatic film was exhibited the publication of Thai patent application number 84089. The hemostatic film fabrication was exposed by using 70-75%w/w rice starch, 5-10%w/w gelatin, 5-10%w/w polyvinyl alcohol, and 10-15%w/w calcium chloride.

The invention of Thai patent application number 84089 had disadvantage that contained gelatin in the composition. Due to gelatin is pseudoproteins, a derivatives of collagen that can be allergic with some patients. In addition, the amide groups of gelatin can be physical cross-linked with the hydroxyl groups of rice starch that decreasing water adsorption. Furthermore, in the preparation of hemostatic film, gelatin powder was dissolved in acetic acid that collaborated in the blood clotting process. The acid involving in a production process was affected to difficult in operation and also to provide acid resistance tools and equipment, especially a freeze dryer that directly affected to a production cost. Another disadvantage of invention, the using of calcium chloridewhich is a cross-linkerwas caused residual ashhigher than the standard values, the US Pharmacopoeia standard values of residual ash must be lower than 2%w/w. Thus the invention of a hemostatic film without the disadvantage mentioned above would be need in this science.

### APPEARANCE AND PURPOSE OF THE INVENTION

The invention related to the composition for preparing surgical hemostatic film rice starch - based was comprised the solutions of gelatinized rice starch, polyvinyl alcohol, carboxymethyl cellulose and glycerol. Moreover, it does not added gelatin, calcium chloride, and also acetic acid.

In addition, this invention was comprised the process for preparing surgical hemostatic film rice starch - based, and/or the surgical hemostatic film rice starch - based that prepared by this process.

The objective of this invention was provided the composition for production a surgical hemostatic film without gelatin, calcium chloride, and acetic acid. This composition was suitably for producing surgical hemostatic film from rice starch.

Another objective of this invention was provided the production process of a surgical hemostatic film from rice starch of the above composition that was a complicated method. And also acid did not use in the process. Thus acid resistant tools did not require.

Another objective of this invention was provided a surgical hemostatic film rice starch - based that is excellent blood adsorption without the substance that caused allergy to the patient and also appropriate price.

### DETAILED DESCRIPTION OF THE INVENTION

The composition for preparing hemostatic film in surgery based on rice starch according to the invention was comprised as following.
a) 4%w/v gelatinized rice starch aqueous solution
b) 10% w/v polyvinyl alcohol aqueous solution
c) 10% w/v carboxymethyl cellulose aqueous solution
d) 99.5% glycerol

Theratios of a): b): c): d) were purposed 100:10:5:1.5, respectively. The gelatinized rice starch was the main composition of the surgical hemostatic film rice starch - based according to the invention. The polyvinyl alcohol was supported the strength of the porous film. The molecular weight of the polyvinyl alcohol was about 85000-124000. The carboxymethyl cellulose was improved the water adsorption capability of the porous film.

The composition of surgical hemostatic film rice starch - based according to the invention does not added gelatin that may be allergic to a patient. And also calcium chloride which is a cross-linker was caused more residual ash that is difficult to control the residual ash after burning according to the standard values. Furthermore, the composition for preparing the surgical hemostatic porous film did not contain acid, and thus tools and equipment of acid resistant did not require.

According to the invention for the production process of above surgical hemostatic film rice starch - based was comprised as following.
a) Preparation of above composition, all solutions were mixed as a homogeneous solution at 60°C for 1 hour.
b) Preparation of surgery hemostatic film was poured in a mold and then frozen at -10 to -20°C for 24 hours.
c) Preparation of surgery hemostatic porous film was done by freeze drying method for 3 days.
d) Sterilization of surgical hemostatic film was done

The sterilization onto a surgical hemostatic film is already known in this science by using gamma ray.

The surgical hemostatic film was casted according to the invention. From the purpose, it can be formed as a cylinder dimension with 10.0 cm diameter and 1.0 cm height.

The surgical hemostatic porous film was prepared according to the invention and the process was not complicated. It also did not use special tools and equipment. Then it can be easily done in industry, and also did not use dangerous solvents or chemicals to the environment.

The surgical hemostatic film rice starch - based according to the invention had interconnected pores about 76% and a diameter size of the porous hold was about 100 µm. It was soft, flexible, water insoluble while it can be degraded *in vivo.* The physical properties of the film can be found in Figure 2. The structure of porous film was observed by using scanning electron microscope (x 3000).

This example was explained the additional invention sample whiles it was not intended to limit the scope of this invention.

### EXPERIMENTAL EXAMPLES

Example 1, surgery hemostatic film rice starch - based as following the invention

Example 1 was prepared by the process according to this invention.
a) The preparation of 4%w/v gelatinized rice starch solution, rice starch powder (Era-Tab, 99%, Erawan Pharmaceutical Research and Laboratory Co., Ltd, Nakorn Pratom, Thailand) was weighed about 4.0 g. After that, it was dissolved in 100 mL distilled water at 80°C, and then stirred with constant speed for 1 hour.
b) The preparation of 10%w/v polyvinyl alcohol solution, polyvinyl alcohol powder (MW 85000-124000, Sigma-Aldrich, USA) was weighed about 10.0 g. After that, it was dissolved in 100 mL distilled water at 90°C, and stirred with constant speed for 2 hours.
c) The preparation of 10%w/v carboxymethyl cellulose solution, carboxymethyl cellulose powder was weighed about 10.0 g. After that, it was dissolved in 100 mL distilled water at 60°C, and then stirred with constant speed for 2 hours.
d) 4%w/v gelatinized rice starch solution, 10%w/v polyvinyl alcohol solution, 10 %w/v carboxymethyl cellulose solution, and glycerol were mixed at the volume ratio of 100:10:5:1.5, respectively. After that, the mixed solution was homogeneously at 60°C, and stirred with constant speed for 1 hour.
e) The homogeneous solution was cooled down at room temperature and then poured into a mold with a cylinder dimension of 10.0 cm diameter and 1.0 cm height. After that, it was frozen in -10 to -20°C for 24 hours.
f) The frozen sample was dried by freeze drying method about 3 days and obtained the surgical hemostatic porous film with a cylinder dimension of 10.0 cm diameter and 1.0 cm height.
g) The sterilization of surgical hemostatic porous film was done by using gamma ray.

Comparison example, surgical hemostatic film rice starch - based according to the publication of Thai patent application number 84089
a) The preparation of 2%w/v gelatinized rice starch solution, rice starch was weighed 2.0 g (Era-Tab, 99%, Erawan Pharmaceutical Research and Laboratory Co., Ltd, Nakorn Pratom, Thailand). After that, it was dissolved in 100 mL distilled water at 60 °C, and then stirred with constant speed at for 1 hour.
b) The preparation of 5%w/v gelatin solution, gelatin powder was weighed about 0.25 g. After that, it was dissolved in 5.0 mL high concentration acetic acid (J.T. Baker, USA) at 100°C, and then stirred with constant speed until transparent gelatin solution appeared.
c) The preparation of 10 %w/v polyvinyl alcohol, the polyvinyl alcohol (MW 77000-82000, Ajax Finechem, Australia) was weighed 10.0 g. After that, it was dissolved in 100 mL distilled water at 90°C, and then stirred at constant speed for 2 hours.
d) The gelatinized rice starch solution was; a) mixed with transparent gelatin solution, and then b) stirred with constant speed at 100 °C about 30 minutes to 1 hour. c) added 2.5 mL of 10%w/v polyvinyl alcohol solution and then heated for 10 minute. Then calcium chloride powder (Ajax, Finechem, Australia) was weighed about 0.5 g. After that, the mixed solution was heated and stirred with constant speed for 10 minutes. Afterwards, a 200 mL of distilled water was added, and then heated and stirred for 2 hours.
h) The homogenous solution was cooled down at room temperature and then poured in a mold with cylinder dimension of 5.75 cm diameter and 1.0 cm height. After that it was frozen at -10 to -20°C for 24 hours.
i) The frozen sample was sublimed by using freeze drying method for 3 days and the porous film with cylinder dimension of 5.75 cm diameter and 0.75 cm height was obtained.
j) The porous film was heated in hot air oven at 50°C for 24 hours. After that, the porous film was heated by using steam at 110°C for 3 hours and then the porous film was heated at 30-40°C until the porous film was dried.
k) The sterilization of surgical hemostatic porous film was done by using gamma ray.

Properties estimation of surgical hemostatic film rice starch - based according to the invention

The surgical hemostatic film rice starch - based according to the invention of example 1 and comparison example were characterized as following.

### Water adsorption determination

The sample 1 or comparison sample of surgical hemostatic film rice starch - based was weighted about 10.0 mg, 3 specimens were separately immersed in 25 mL distilled water for 30 seconds. After that, the swollen sample was removed and reweighed. The water adsorption was calculated from the swollen weight compared with initial weight and the experiment was repeated 3 times.

### pH measurement

The sample 1 or comparison sample of surgical hemostatic film rice starch - based was weighed about 50.0 mg, 3 specimens were separately immersed in 2 mL of distilled water for 1 minute. After that, the sample was removed from distilled water. The pH measurement was tested from the residual distilled water.

### Residual ash after ignition examination

The sample 1 or comparison sample of surgical hemostatic film was weighed about 50.0 mg, 3 specimens were separately heated in crucible by flame. The residual ash was weighed and then calculated the percentage of residual ash by residual weight compared with initial weight.

**Table 1 Properties comparison of surgery hemostatic film rice starch - based according to this invention and the advertisement of Thai patent application number 84089**

| Properties | This invention sample | Comparison sample |
|---|---|---|
| water adsorption | 13.40 times | 11.29 times |
| average pH | 6.5 | 4.6 |
| percentage of residual ash | 0.28% | 0.40% |

From Table 1, the water adsorption of surgery hemostatic film rice starch - based of this invention was higher than the previously invention. That revealed the more ability of blood adsorption. Moreover, the residual ash after ignition was also lower than the previously invention.

### BEST PROCEDURE OF THE INVENTION

Similarly as the detailed description of the invention.

## Claims

1. The composition for preparing surgical hemostatic film rice starch - based was comprised as following.
a) 4 %w/v gelatinized rice starch aqueous solution
b) 10 %w/v polyvinyl alcohol aqueous solution
c) 10 %w/v carboxymethyl cellulose aqueous solution
d) Glycerol

2. The composition for preparing surgical hemostatic film rice starch - based of claim 1, the ratios of a): b): c): d) were 100:10:5:1.5, respectively.

3. A process for preparing surgical hemostatic film rice starch - based that comprises a procedure as following:
a) The preparation of composition of claim 1 or 2 by mixing solutions as homogeneous solution at 60°C for 1 hour,
b) The casting of surgical hemostatic film was poured in a mold and then frozen at -10 to -20°C for 24 hours,
c) The preparation of surgical hemostatic porous film was done by freeze drying method for 3 days,
d) The sterilization of surgical hemostatic film was done.

4. The process according to claim 3,
**characterized in,**
**that** the procedure for sterilization on the hemostatic film was gamma ray.

5. Surgical hemostatic film rice starch - based was prepared from composition of claim 1 or 2.

## Patentansprüche

1. Zusammensetzung zur Herstellung eines chirurgischen hämostatischen Films auf Reisstärkebasis aufweisend
a) 4% m/v gelatinierte wässrige Reisstärkelösung
b) 10% m/v wässrige Polyvinylalkohollösung
c) 10% m/v wässrige Carboxymethylcelluloselösung
d) Glycerin

2. Zusammensetzung zur Herstellung von chirurgischer hämostatischer Filmreisstärke nach Anspruch 1, wobei die Verhältnisse von a): b): c): d) 100: 10: 5: 1,5 ist.

3. Verfahren zur Herstellung eines chirurgischen hämostatischen Films auf Reisstärkebasis, mit den folgenden Schritten:
a) Herstellung der Zusammensetzung nach Anspruch 1 oder 2 durch Mischen von Lösungen als homogene Lösung bei 60 ° C für 1 Stunde,
b) chirurgischen hämostatischen Films wird in eine Form gegossen und dann 24 Stunden bei -10 bis -20°C eingefroren,
c) Herstellung des chirurgischen hämostatischen porösen Films erfolgt durch ein Gefriertrocknungsverfahren für 3 Tage,
d) Sterilisation des chirurgischen hämostatischen Films wird durchgeführt.

4. Verfahren nach Anspruch 3,
**dadurch gekennzeichnet,**
**dass** Sterilisation des hämostatischen Film mittels Gammastrahlung erfolgt.

5. Ein chirurgischer hämostatischer Film auf Reisstärkebasis basierend auf der Zusammensetzung nach Anspruch 1 oder 2.

## Revendications

1. Composition pour la production d'un filme chirurgien hémostatique à base d'amidon de riz présentant
a) 4% m/v d'une solution d'amidon de riz gélatinée aqueuse
b) 10% m/v d'une solution aqueuse d'alcool polyvinylique
c) 10% m/v d'une solution aqueuse de carboxyméthylcellulose
d) Glycérol

2. Composition pour la production d'amidon de riz de filme chirurgien hémostatique selon la revendication 1 où les rapports se situent à a): b): c): d) 100: 10: 5: 1,5.

3. Procédé pour la production d'un filme chirurgien hémostatique sur la base d'amidon de riz présentant les étapes qui suivent:
a) Production de la composition selon la revendication 1 ou 2 par le biais du mixage de solutions formant une solution homogène à 60 ° C pour une 1 heure,
b) Le filme chirurgien hémostatique est moulé dans une forme et gelé par la suite 24 heures à -10 jusqu'à -20°C,
c) Production d'un filme chirurgien hémostatique poreux a lieu par le biais d'un procédé de lyophilisation pour 3 jours,
d) Stérilisation du filme chirurgien hémostatique est effectuée.

4. Procédé selon la revendication 3 est caractérisé de sorte que la stérilisation du filme hémostatique est réalisée grâce au rayonnement Gamma.

5. Un filme chirurgien hémostatique sur la base d'amidon de riz se basant sur la composition selon la revendication 1 ou 2.
